# EUROPÄISCHE PATENTSCHRIFT

(11) **EP 1 318 407 B1**
(45) Veröffentlichungstag und Bekanntmachung des Hinweises auf die Patenterteilung: **29.09.2004**
(21) Anmeldenummer: 01129121.8
(22) Anmeldetag: 07.12.2001
(51) Int. Cl.: G01N 33/68, G01N 33/569, A61P 31/04, A61P 37/02

(54) **Verwendungen der Aldose-1-Epimerase (Mutarotase) für die Diagnose von Entzündungserkrankungen und Sepsis**
Use of aldose-1-epimerase (mutarotase) for the diagnosis of infections and sepsis
Utilisation de l'aldose-1-epimerase (mutarotase) pour le diagnostic des infections et de la septicémie

(43) Veröffentlichungstag der Anmeldung: 11.06.2003
(73) Patentinhaber: B.R.A.H.M.S Aktiengesellschaft, 16761 Hennigsdorf (DE)
(72) Erfinder: Bergmann, Andreas, Dr., 12351 Berlin (DE); Struck, Joachim, Dr., 12161 Berlin (DE); Ühlein, Monika, Dr., 10407 Berlin (DE)
(74) Vertreter: Andrae, Steffen, Dr.

(56) Entgegenhaltungen:
- GABAY CEM ET AL: "Acute-phase proteins and other systemic responses to inflammation." NEW ENGLAND JOURNAL OF MEDICINE, Bd. 340, Nr. 6, 11. Februar 1999 (1999-02-11), Seiten 448-454, XP008003139 ISSN: 0028-4793
- WEIBEL M K: "A COUPLED ENZYME ASSAY FOR ALDOSE 1 EPIMERASE EC-5.1.3.3" ANALYTICAL BIOCHEMISTRY, Bd. 70, Nr. 2, 1976, Seiten 489-494, XP008003394 ISSN: 0003-2697
- DATABASE EMBL [Online] 1. Dezember 2001 (2001-12-01) STRAUSBERG, R.: "Hypothetical 37,8 kDA Protein" Database accession no. Q96C23 XP002199481
- REINHART, K ET AL: "Intensivmedizin: Sepsis und septischer Schock (Seiten 756-760)" 2001 , THIEME VERLAG XP008003130 * Seite 756, rechte Spalte, Absatz 3 *
- BEISHUIZEN A ET AL: "Endogenous mediators in sepsis and septic shock" ADVANCES IN CLINICAL CHEMISTRY, Bd. 33, 1999, Seiten 55-131, XP008003176

### SEQUENCE LISTING

<110> B.R.A.H.M.S Aktiengesellschaft
<120> Verwendungen der Aldose-1-Epimerase (Mutarotase) für die Diagnose und Therapie von Entzündungserkrankungen und Sepsis
<130> 3684 AS
<140>
<141>
<160> 3
<170> PatentIn Ver. 2.1
<210> 1
<211> 6
<212> PRT
<213> Primat (Pavian)
<400> 1
   Phe Gly Glu Leu Pro Ser
     1               5
<210> 2
<211> 7
<212> PRT
<213> Primat (Pavian)
<400> 2
   Pro Asp Gly Glu Glu Gly Tyr
    1               5
<210> 3
<211> 342
<212> PRT
<213> Homo sapiens
<400> 3
   Met Ala Ser Val Thr Arg Ala Val Phe Gly Glu Leu Pro Ser Gly Gly
    1               5                  10                  15
   Gly Thr Val Glu Lys Phe Gln Leu Gln Ser Asp Leu Leu Arg Val Asp
               20                  25                  30
   Ile Ile Ser Trp Gly Cys Thr Ile Thr Ala Leu Glu Val Lys Asp Arg
           35                  40                  45
   Gln Gly Arg Ala Ser Asp Val Val Leu Gly Phe Ala Glu Leu Glu Gly
       50                  55                  60
   Tyr Leu Gln Lys Gln Pro Tyr Phe Gly Ala Val Ile Gly Arg Val Ala
   65                  70                  75                  80
   Asn Arg Ile Ala Lys Gly Thr Phe Lys Val Asp Gly Lys Glu Tyr His
                   85                  90                  95
   Leu Ala Ile Asn Lys Glu Pro Asn Ser Leu His Gly Gly Val Arg Gly
               100                 105                 110
   Phe Asp Lys Val Leu Trp Thr Pro Arg Val Leu Ser Asn Gly Val Gln
           115                 120                 125
   Phe Ser Arg Ile Ser Pro Asp Gly Glu Glu Gly Tyr Pro Gly Glu Leu
       130                 135                 140
   Lys Val Trp Val Thr Tyr Thr Leu Asp Gly Gly Glu Leu Ile Val Asn
   145                 150                 155                 160
   Tyr Arg Ala Gln Ala Ser Gln Ala Thr Pro Val Asn Leu Thr Asn His
                   165                 170                 175
   Ser Tyr Phe Asn Leu Ala Gly Gln Ala Ser Pro Asn Ile Asn Asp His
               180                 185                 190
   Glu Val Thr Ile Glu Ala Asp Thr Tyr Leu Pro Val Asp Glu Thr Leu
           195                 200                 205
   Ile Pro Thr Gly Glu Val Ala Pro Val Gln Gly Thr Ala Phe Asp Leu
       210                 215                 220
   Arg Lys Pro Val Glu Leu Gly Lys His Leu Gln Asp Phe His Leu Asn
   225                 230                 235                 240
   Gly Phe Asp His Asn Phe Cys Leu Lys Gly Ser Lys Glu Lys His Phe
                   245                 250                 255
   Cys Ala Arg Val His His Ala Ala Ser Gly Arg Val Leu Glu Val Tyr
               260                 265                 270
   Thr Thr Gln Pro Gly Val Gln Phe Tyr Thr Gly Asn Phe Leu Asp Gly
           275                 280                 285
   Thr Leu Lys Gly Lys Asn Gly Ala Val Tyr Pro Lys His Ser Gly Phe
       290                 295                 300
   Cys Leu Glu Thr Gln Asn Trp Pro Asp Ala Val Asn Gln Pro Arg Phe
   305                 310                 315                 320
   Pro Pro Val Leu Leu Arg Pro Gly Glu Glu Tyr Asp His Thr Thr Trp
                   325                 330                 335
   Phe Lys Phe Ser Val Ala
               340

## Beschreibung

Die vorliegende Erfindung betrifft neue Verwendungen des Enzyms Aldose-1-Epimerase (auch bekannt als Mutarotase; nachfolgend häufig abgekürzt als "A1E" oder einfach "Epimerase") für die medizinischen Diagnostik von Entzündungserkrankungen und Sepsis. Sie beruht auf dem erstmaligen Nachweis stark erhöhter Konzentrationen der Aldose-1-Epimerase in Lebergewebe von Primaten, bei denen experimentell durch Toxinverabreichunge eine Sepsis bzw. systemische Entzündung erzeugt worden war.

Die vorliegende Erfindung hat ihren Ursprung in intensiven Forschungsarbeiten der Anmelderin im Zusammenhang mit weiteren Verbesserungen der Diagnose und Therapie von Entzündungen und Infektionen, insbesondere von Entzündungen infektiöser Ätiologie und Sepsis.

Als Entzündungen (Inflammationen) werden ganz allgemein bestimmte physiologische Reaktionen eines Organismus auf verschiedenartige äußere Einwirkungen wie z.B. Verletzungen, Verbrennungen, Allergene, Infektionen durch Mikroorganismen wie Bakterien und Pilze und Viren, auf Fremdgewebe, die Abstoßungsreaktionen auslösen, oder auf bestimmte entzündungsauslösende endogene Zustände des Körpers, z.B. bei Autoimmunerkrankungen und Krebs, bezeichnet. Entzündungen können als harmlose, lokal begrenzte Reaktionen des Körpers auftreten, sind jedoch auch typische Merkmale zahlreicher ernster chronischer und akuter Erkrankungen von einzelnen Geweben, Organen, Organ- und Gewebsteilen.

Lokale Entzündungen sind dabei meist Teil der gesunden Immunreaktion des Körpers auf schädliche Einwirkungen, und damit Teil des lebenserhaltenden Abwehrmechanismus des Organismus. Wenn Entzündungen jedoch Teil einer fehlgeleiteten Reaktion des Körpers auf bestimmte endogene Vorgänge wie z.B. bei Autoimmunerkrankungen sind und/oder chronischer Natur sind, oder wenn sie systemische Ausmaße erreichen, wie beim systemischen Inflammationssyndrom (Systemic Inflammatory Response Syndrome, SIRS) oder bei einer auf infektiöse Ursachen zurückzuführenden schweren Sepsis, geraten die für Entzündungsreaktionen typischen physiologischen Vorgänge außer Kontrolle und werden zum eigentlichen, häufig lebensbedrohlichen Krankheitsgeschehen.

Es ist heute bekannt, dass die Entstehung und der Verlauf von entzündlichen Prozessen von einer beträchtlichen Anzahl von Substanzen, die überwiegend proteinischer bzw. peptidischer Natur sind, gesteuert werden bzw. von einem mehr oder weniger zeitlich begrenzten Auftreten bestimmter Biomoleküle begleitet sind. Zu den an Entzündungsreaktionen beteiligten endogenen Substanzen gehören insbesondere solche, die zu den Cytokinen, Mediatoren, vasoaktiven Substanzen, Akutphasenproteinen und/oder hormonellen Regulatoren gezählt werden können. Die Entzündungsreaktion stellt eine komplexe physiologische Reaktion dar, an der sowohl das Entzündungsgeschehen aktivierende endogene Substanzen (z.B. TNF-α) als auch desaktivierende Substanzen (z.B. Interleukin-10) beteiligt sind.

Bei systemischen Entzündungen wie im Falle einer Sepsis bzw. des septischen Schocks weiten sich die entzündungsspezifischen Reaktionskaskaden unkontrolliert auf den gesamten Körper aus und werden dabei, im Sinne einer überschießenden Immunantwort, lebensbedrohlich. Zu den gegenwärtigen Kenntnissen über das Auftreten und die möglichen Rolle einzelner Gruppen endogener entzündungsspezifischer Substanzen wird beispielsweise verwiesen auf A.Beishuizen, et al., "Endogenous Mediators in Sepsis and Septic Shock", Advances in Clinical Chemistry, Vol.33, 1999, 55-131; und C.Gabay, et al., "Acute Phase Proteins and Other Systemic Responses to Inflammation", The New England Journal of Medicine, Vol.340, No.6, 1999, 448-454. Da sich das Verständnis von Sepsis und verwandten systemischen entzündlichen Erkrankungen, und damit auch die anerkannten Definitionen, in den letzten Jahren gewandelt haben, wird außerdem verwiesen auf K.Reinhart, et al., "Sepsis und septischer Schock", in: Intensivmedizin, Georg Thieme Verlag, Stuttgart.New York, 2001, 756-760; wo eine moderne Definition des Sepsis-Begriffes gegeben wird. Im Rahmen der vorliegenden Anmeldung werden die Begriffe Sepsis bzw. entzündliche Erkrankungen in Anlehnung an die Definitionen verwendet, wie sie den genannten drei Literaturstellen entnommen werden können.

Während wenigstens im europäischen Raum die durch eine positive Blutkultur nachweisbare systemische bakterielle Infektion lange den Sepsisbegriff prägte, wird die Sepsis heute in erster Linie als systemische Entzündung verstanden, die infektiöse Ursachen hat, als Krankheitsgeschehen jedoch große Ähnlichkeiten mit systemischen Entzündungen aufweist, die durch andere Ursachen ausgelöst werden. Dem genannten Wandel des Sepsis-Verständnisses entsprechen Veränderungen der diagnostischen Ansätze. So wurde der direkte Nachweis bakterieller Erreger durch komplexe Überwachungen physiologischer Parameter und in jüngerer Zeit insbesondere auch durch den Nachweis bestimmter am Sepsisgeschehen bzw. am Entzündungsgeschehen beteiligter endogener Substanzen, d.h. spezifischer "Biomarker", ersetzt bzw. ergänzt.

Von der großen Zahl von Mediatoren und Akutphasenproteinen, von denen man weiß, dass sie an einem Entzündungsgeschehen beteiligt sind, eignen sich dabei für diagnostische Zwecke insbesondere solche, deren Auftreten sehr spezifisch für entzündliche Erkrankungen bzw. bestimmte Phasen entzündlicher Erkrankungen ist, deren Konzentrationen sich drastisch und diagnostisch signifikant verändern und die außerdem die für Routinebestimmungen erforderlichen Stabilitäten aufweisen und hohe Konzentrationswerte erreichen. Für diagnostische Zwecke steht dabei die zuverlässige Korrelation von Krankheitsgeschehen (Entzündung, Sepsis) mit dem jeweiligen Biomarker im Vordergrund, ohne dass dessen Rolle in der komplexen Kaskade der am Entzündungsgeschehen beteiligten endogenen Substanzen bekannt sein muss.

Eine derartige als Sepsis-Biomarker besonders geeignete endogene Substanz ist Procalcitonin. Procalcitonin ist ein Prohormon, dessen Serum-Konzentrationen unter den Bedingungen einer systemischen Entzündung infektiöser Ätiologie (Sepsis) sehr hohe Werte erreichen, während es bei Gesunden so gut wie nicht nachweisbar ist. Hohe Werte an Procalcitonin werden außerdem in einem relativ frühen Stadium einer Sepsis erreicht, so dass sich die Bestimmung von Procalcitonin auch zu Früherkennung einer Sepsis und zur frühen Unterscheidung einer infektiös bedingten Sepsis von schweren Entzündungen, die auf anderen Ursachen beruhen, eignet. Die Bestimmung von Procalcitonin als Sepsismarker ist Gegenstand der Veröffentlichung M.Assicot, et al., "High serum procalcitonin concentrations in patients with sepsis and infection", The Lancet, vol.341, No.8844, 1993, 515-518; und der Patente DE 42 27 454 C2 bzw. EP 0 656 121 B1 bzw. US 5,639,617. Auf die genannten Patente und in der genannten Veröffentlichung angeführten frühen Literaturstellen wird zur Ergänzung der vorliegenden Beschreibung ausdrücklich Bezug genommen. In den letzten Jahren ist die Zahl der Veröffentlichungen zum Thema Procalcitonin stark angestiegen. Stellvertretend für zusammenfassende Veröffentlichungen aus jüngerer Zeit wird daher noch verwiesen auf W.Karzai et al., "Procalcitonin - A New Indicator of the Systemic Response to Severe infection", Infection, Vol. 25, 1997, 329-334; und M.Oczenski et al., "Procalcitonin: a new parameter for the diagnosis of bacterial infection in the peri-operative period", European Journal of Anaesthesiology 1998, 15, 202-209; sowie ferner H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", in: Sepsis 1998; 2:243-253; und die darin zitierten weiteren Literaturstellen.

Die Verfügbarkeit des Sepsismarkers Procalcitonin hat der Sepsisforschung starke Impulse gegeben, und es werden gegenwärtig intensive Anstrengungen unternommen, weitere Biomarker zu finden, die die Procalcitonin-Bestimmung ergänzen können und/oder zusätzliche Informationen für Zwecke der Feindiagnostik bzw. Differentialdiagnostik zu liefern vermögen. Erschwert wird die Suche nach potentiellen neuen Sepsis-Biomarkern allerdings dadurch, dass häufig noch sehr wenig oder nichts über die genaue Funktion bzw. über die genauen Gründe für das Auftreten bestimmter endogener Substanzen, die am Entzündungs- oder Sepsisgeschehen beteiligt sind, bekannt ist.

Die Ergebnisse der experimentellen Überprüfung eines fruchtbaren rein hypothetischen Ansatzes zur Ermittlung weiterer potentieller Sepsismarker finden sich in DE 198 47 690 A1 bzw. WO 00/22439. Dort wird gezeigt, dass bei Sepsis nicht nur die Konzentration des Prohormons Procalcitonin erhöht ist, sondern auch für andere Substanzen, die zu den Peptid-Prohormonen gerechnet werden können, signifikant erhöhte Konzentrationen beobachtet werden können. Während das beschriebene Phänomen gut dokumentiert ist, sind die Ursachen für den Anstieg der Konzentrationen von Prohormonen bei Sepsis weiterhin weitgehend ungeklärt.

In der vorliegenden Anmeldung wird ein Ergebnis eines anderen fruchtbaren, rein experimentellen Ansatzes für die Suche nach weiteren entzündungs- bzw. sepsisspezifischen Biomolekülen berichtet. Auch diese experimentellen Untersuchungen nehmen ihren Ausgang bei der Bestimmung von Procalcitin im Zusammenhang mit systemischen entzündlichen Reaktionen infektiöser Ätiologie. So war sehr früh beobachtet worden, dass bei Sepsis das Procalcitonin offensichtlich nicht auf die gleiche Weise gebildet wird, wie dann, wenn es Vorläufer für das Hormon Calcitonin ist. So wurden hohe Procalcitoninspiegel auch bei Patienten beobachtet, denen die Schilddrüse entfernt worden war. Deshalb kann die Schilddrüse nicht dasjenige Organ sein, in dem Procalcitonin bei Sepsis gebildet bzw. ausgeschüttet wird. In den Veröffentlichungen H.Redl, et al., "Procalcitonin release patterns in a baboon model of trauma and sepsis: Relationship to cytokines and neopterin", Crit Care Med 2000, Vol.28. No.11, 3659-3663; und H.Redl, et al., "Non-Human Primate Models of Sepsis", Sepsis 1998; 2:243-253; werden die Ergebnissen von experimentellen Untersuchungen berichtet, die der Klärung der Bildung von Procalcitonin bei Sepsis dienen sollten. In den genannten Arbeiten wird durch Endotoxinverabreichung an Primaten (Paviane) eine künstliche Sepsis erzeugt, und es wird bestimmt, bei welchen experimentell erzeugten Zuständen die höchsten Procalcitoninkonzentrationen im Blut erreicht werden. Eine Weiterentwicklung des in den genannten Arbeiten beschriebene Versuchstiermodells dient im Rahmen der vorliegenden Anmeldung dazu, neue endogene sepsisspezifische Biomarker von peptischer bzw. proteinischer Natur zu ermitteln, deren Auftreten für Sepsis oder bestimmte Formen von Sepsis charakteristisch ist und die daher eine spezifische Sepsisdiagnose ermöglichen. Das Primatenmodell wurde dabei aufgrund der sehr großen Ähnlichkeit der Physiologie von Primaten und Menschen und der hohen Kreuzreaktivität mit vielen therapeutischen und diagnostischen humanen Reagenzien gewählt.

Da die bei Entzündungen gebildeten endogenen Substanzen Teil der komplexen Reaktionskaskade des Körpers sind, sind derartige Substanzen außerdem nicht nur von diagnostischem Interesse, sondern es wird gegenwärtig auch mit erheblichem Aufwand versucht, durch Beeinflussung der Entstehung und/oder der Konzentration einzelner derartiger Substanzen therapeutisch in das Entzündungsgeschehen einzugreifen, um die zum Beispiel bei Sepsis beobachtete systemische Ausbreitung der Entzündung möglichst frühzeitig zu stoppen. In diesem Sinne sind nachweislich am Entzündungsgeschehen beteiligte endogene Substanzen auch als potentielle therapeutische Targets anzusehen. Versuche, ansetzend an bestimmten Mediatoren des Entzündungsgeschehens dieses positiv therapeutisch zu beeinflussen, sind beschrieben beispielsweise in E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170; oder K.Garber, "Protein C may be sepsis solution", Nature Biotechnology, Vol.18, 2000, 917-918. Diese therapeutischen Ansätze laufen darauf hinaus, die Konzentrationen entzündungsfördernder Substanzen zu senken bzw. die Entstehung derartiger Substanzen zu hemmen, und zwar insbesondere unter Verwendung von spezifischen Antikörpern (gegen TNF-α bzw. MIF; vgl. E.A.Panacek, "Anti-TNF strategies", Journal für Anästhesie und Intensivbehandlung; Nr.2, 2001, 4-5; T.Calandra, et al., "Protection from septic shock by neutralization of macrophage migration inhibitory factor", Nature Medicine, Vol.6, No.2, 2000, 164-170) bzw. die Konzentration von hemmend in die Entzündungskaskade eingreifenden endogenen Substanzen (Protein C; K.Garber, "Protein C may be sepsis solution", Nature Biotechnology, Vol.18, 2000, 917-918) zu erhöhen. In der letztgenannten Veröffentlichung findet sich ein Überblick über derartige, leider bisher meist wenig erfolgreiche Versuche, das Entzündungsgeschehen unter Beeinflussung ausgewählter endogener Target-Moleküle therapeutisch zu beeinflussen. Angesichts der bisherigen eher enttäuschenden therapeutischen Ansätze besteht ein hohes Interesse daran, weitere möglichst entzündungs- bzw. sepsisspezifische endogene Biomoleküle zu identifizieren, die auch als therapeutische Targets neue Erfolgsaussichten für die Entzündungsbekämpfung eröffnen.

Die vorliegenden Erfindung beruht darauf, dass sich in Primaten und Menschen bei infektiös bedingten Entzündungen das Enzym Aldose-1-Epimerase (Mutarotase) in beträchtlichen Konzentrationen nachweisen läßt, und zwar im Unterschied zu Gesunden, bei denen es nicht oder nur in Konzentrationen an der analytischen Nachweisgrenze gefunden wird, was die genannte Epimerase sowohl für die Entzündungsdiagnostik/-Sepsisdiagnosik als auch als neues therapeutisches Target geeignet macht.

Die Verwendung in der Diagnose die sich aufgrund des erstmals nachgewiesenen Auftretens der Aldose-1-Epimerase bei der experimentellen Simulation von Entzündungen bzw. Sepsis ergeben, wird in allgemeiner Form in Anspruch 1 beansprucht.

Die Ansprüche 2 bis 9 betreffen dabei diagnostische Verwendungen bzw. Verfahren.

Wie nachfolgend im experimentellen Teil noch näher ausgeführt wird, beruht die Erfindung darauf, dass nach experimenteller Auslösung einer künstlichen Sepsis in Pavianen durch Endotoxinverabreichung (LPS aus Salmonella Typhimurium) und 2D-gelektrophoretischer Aufarbeitung von Lebergewebe der behandelten Tiere ein nur bei den behandelten Tieren identifizierbares Peptid- bzw. Proteinprodukt gefunden werden konnte. Dieses spezifische Produkt wurde aus dem Elektrophoresegel isoliert, trypsinverdaut und massenspektrometrisch untersucht. Es erwies sich, auf der Basis der Identifizierung zweier selektierter und durch Tandem-Massenspektrometrie genauer charakterisierter Peaks, die einem in einer humanen cDNA-Datenbank zu findenden hypothetischen Protein zugeordnet werden konnten, und die nachfolgende Identifizierung dieses Proteins durch Vergleich mit den Daten für ein humanes Aldose-1-Epimerase-Fragment bzw. für die Aldose-1-Epimerase aus Schweinen, als Aldose-1-Epimerase (Pavian).

Aufgrund der Identität der massenspektrometrisch identifizierten Sequenzen mit Teilsequenzen der in Datenbanken zu finden Sequenzdaten zur Aldose-1-Epimerase ist die Identifizierung des humanen Äquivalents zu dem isolierten Proteinspot als Aldose-1-Epimerase nach anerkannten Kriterien als eindeutig anzusehen.

Wenn in der vorliegenden Anmeldung das für diagnostische Zwecke bestimmte vorgeschlagene Peptid als "Epimerase" bezeichnet wird, bedeutet das nicht, das eine solche Epimerase eine 100%ige Identität mit der Sequenz gemäss SEQ ID NO:3 aufweisen muss. Als Epimerase wird in der vorliegenden Anmeldung vielmehr ein Peptid bezeichnet, das in der physiologisch vorkommenden Form Peptid-Teilsequenzen gemäss SEQ ID NO:1 (FGELPS) und SEQ ID:NO2 (PDGEEGY) aufweist, und eine hohe Homologie, d.h. vorzugsweise von mehr als 60%, stärker bevorzugt 80%, zu der Sequenz der humanen Epimerase gemäß SEQ ID NO:3 aufweist.

Von dem Begriff Epimerase werden auch Teilpeptide eines wie oben definierten Peptids erfasst, die insbesondere bei der Herstellung von Reagenzien, z.B. selektiven Antikörpern, für die Epimerase-Bestimmung bzw. zur Herstellung von Assays zur Epimerase-Bestimmung in biologischen Proben verwendet werden können. Als Epimerase bzw. Teilsequenzen davon sind auch solche Sequenzen anzusehen, die nach Deletion von einer oder mehreren Aminosäuren oder kurzer Peptidsequenzen aus dem Peptid gemäss SEQ ID NO:3 erhalten werden. Ferner sind für diagnostische Zwecke geeignete Teilsequenzen (Fragmente) insbesondere solche, die eine Folge von mindestens drei Aminosäuren, vorzugsweise mindestens 6 Aminosäuren, des Peptids SEQ ID NO:3 umfassen.

Für besondere diagnostische Zwecke können die erfindungsgemäßen Epimerase-Peptide auch tierische Peptide, insbesondere Säugetierpeptide, sein, die eine wenigstens 60%ige Homologie zu dem Peptid mit SEQ ID NO:3 aufweisen sollten und z.B. für diagnostische Zwecke oder in der Veterinärmedizin verwendet werden können.

Die nach anerkannten Kriterien als eindeutig anzusehende Identifizierung des nur nach Sepsis- bzw. Entzündungsauslösung in dem Pavian-Lebergewebe gefundenen Proteins als Aldose-1-Epimerase (Mutarotase; A1E; EC 5.1.3.3.) ist von hohem wissenschaftlichen, diagnostischen und therapeutischen Interesse. Als enzymatische Gewebe-Aktivität ist die Epimerase schon lange bekannt. Eine Identifizierung der in der Datenbank ermittelten humanen cDNA-Sequenz als eine für die vollständige humane Aldose-1-Epimerase kodierende Sequenz ist nach diesseitiger Kenntnis noch nicht erfolgt. Die Epimerase-Aktivität war bisher Gegenstand von Untersuchungen mit primär wissenschaftlicher Zielsetzung. Es können hier beispielsweise genannt werden Veröffentlichungen von Michael K. Weibel in: Analytical Biochemistry 70, 489-494 (1976); oder Jane A. Beebe et al., in: Biochemistry 1998, 37, 14989-14997 (zur Galactose-Mutarotase), und die darin genannten Veröffentlichungen. In den genannten Arbeiten werden Epimerasen in erster Linie als Enzyme diskutiert, die die gegenseitige Umwandlung von α- und β-Anomeren von Aldosen katalysieren und für die angenommen wird, dass sie am Zucker-Transport, z.B. in Niere und Darm, und am Zuckerstoffwechsel beteiligte sind. Für die medizinische Diagnostik und Therapie spielte die Aldose-1-Epimerase bisher keine Rolle.

Mit der vorliegenden Erfindung wird einerseits, gemäß Anspruch 1, Schutz für die darin genannten Verwendungen eines Peptids begehrt, das als Epimerase bezeichnet wird und gemäss der o.g. Definition primär durch die Anwesenheit der Teilsequenzen aus 6 bzw. 7 Aminosäuren gemäß SEQ ID NO:1 und SEQ ID:NO2 definiert ist, ohne dass bezüglich der Länge und Natur weiterer Aminosäuresequenzen Einschränkungen bestehen sollen. Durch die Anwesenheit der genannten beiden Teilsequenzen ist die humane A1E von allen anderen bekannten und/oder in Datenbanken dokumentierten humanen Peptiden und Proteinen unterscheidbar. In der vollständigen humanen Form weist die humane Aldose-1-Epimerase die Sequenz gemäss SEQ ID NO:3 auf.

Aufgrund der nunmehr bekannten Sequenz SEQ ID NO:3 und der physiologischen Rolle der humanen Epimerase in Verbindung mit den Befunden zu ihrer verstärkten Bildung bei Entzündungen und Sepsis können die humane Epimerase bzw. Teile davon für diagnostische und/oder therapeutische Zwecke nach Verfahren, die inzwischen zum Stand der Technik gehören, synthetisch oder gentechnologisch als Rekombinationsprodukte hergestellt werden.

Ferner können die Epimerase-Peptide nach bekannten Verfahren des modernen Standes der Technik auch zur Erzeugung spezifischer polyklonaler und insbesondere monoklonaler Antikörper verwendet werden, die als Hilfsmittel für die diagnostische Bestimmung der erfindungsgemäßen Peptide und/oder auch als potentielle therapeutische Mittel geeignet sind. Die Erzeugung geeigneter monoklonaler Antikörper gegen bekannte Peptid-Teilsequenzen gehört heute zum allgemeinen Stand der Technik.

Bei der medizinisch-diagnostischen Bestimmung der Epimerase gemäss SEQ ID NO:3 oder von ausgewählten Teilpeptiden davon kann dabei grundsätzlich so vorgegangen werden, wie das z.B. für die selektive Procalcitoninbestimmung beschrieben ist in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; wobei ausdrücklich ergänzend auch auf die dort beschriebenen Immunisierungstechniken verwiesen wird, die eine Möglichkeit für die Gewinnung von monoklonalen Antikörpern auch gegen Teilsequenzen der Epimerase darstellen. Variationen der beschriebenen Techniken und/oder weitere Immunisierungstechniken kann der Fachmann einschlägigen Standardwerken und Veröffentlichungen entnehmen und sinngemäß anwenden.

Ferner ist auch ausdrücklich die Erzeugung von Epimerase-Antikörpern unter Anwendung von Techniken der direkten genetischen Immunisierung mit DNA zu erwähnen. Es liegt ferner im Rahmen der vorliegenden Erfindung, zur Immunisierung z.B. eine cDNA der gewünschten Epimerase oder ihrer Teilpeptide zu verwenden, da es sich in der Vergangenheit gezeigt hat, dass durch derartige Immunisierungstechniken das Spektrum der gewinnbaren Antikörper erweitert werden kann. Epimerase gemäß SEQ ID NO:3 oder Teilpeptide davon, z.B. solche, die die Teilsequenz SEQ ID NO:1, SEQ ID NO:2 und/oder andere Teilsequenzen enthalten, können aufgrund der vorliegenden Ergebnisse als spezifische Markerpeptide (Biomarker) zum diagnostischen Nachweis und zur Verlaufskontrolle von Entzündungen und Infektionen (insbesondere auch von systemischen Infektionen vom Sepsistyp) dienen. Wie die Bestimmung von Procalcitonin kann dabei die Bestimmung der Epimerase durch die Anwendung eines Verfahrens zur differentialdiagnostischen Früherkennung und zur Erkennung sowie zur Beurteilung des Schweregrads und zur therapiebegleitenden Verlaufsbeurteilung von Sepsis und Infektionen erfolgen, wobei man bei einem derartigen Verfahren in einer Probe einer biologischen Flüssigkeit oder eines Gewebes eines Patienten den Gehalt an Epimerase oder eines Teilpeptids davon bestimmt und aus der festgestellten Anwesenheit und/oder Menge des bestimmten Peptids auf das Vorliegen einer Entzündung, einer schweren Infektion oder einer Sepsis schließt und das erhaltene Ergebnis mit dem Schweregrad der Sepsis korreliert und die Behandlungsmöglichkeiten und/oder die Behandlungsaussichten abschätzt.

An Stelle der Bestimmung der Epimerase oder ihrer Bruchstücke oder ggf. von posttranslational modifizierten Formen davon ist für diagnostische Zwecke auch die Bestimmung der zugehörigen A1E-mRNA möglich. Die Epimerase-Bestimmung kann für diagnostische Zwecke auch indirekt als Bestimmung ihrer Enzymaktivität in einem entzündeten Organ oder Gewebe oder einer biologischen Flüssigkeit erfolgen.

Es ist ferner möglich, die Bestimmung von Epimerase als Prognosemarker und Marker für die Überwachung der Krankheitsverlaufs von Entzündungen, insbesondere systemischen Entzündungen, und Sepsis im Rahmen einer Kombinationsmessung mit anderen Markern durchzuführen.

Neben einer Kombination mit einer Procalcitoninmessung kommt insbesondere eine Kombination der Messung von Epimerase mit der Bestimmung anderer Marker für Sepsis und systemische Entzündungen in Betracht, und zwar insbesondere mit CA 19-9, CA 125, S100B oder an der Regulierung von Entzündungen beteiligten S100A-Proteinen, oder mit der Bestimmung der in den älteren, nachfolgend genannten deutschen Patentanmeldungen der Anmelderin beschriebenen neuartigen Sepsismarker Inflammin (DE 101 19 804.3) und CHP (DE 101 31 922.3), der Bestimmung des Enzyms Glycin-N-Acyltransferase (GNAT) und/oder mit der Bestimmung von löslichen Cytokeratinfragmenten, insbesondere der neu aufgefundenen löslichen Cytokeratin-1-Fragmente (sCY1F;; DE 101 30 985.6) sowie der bekannten Tumormarker CYFRA-21 oder TPS und/oder eines oder mehrerer der o.g. Prohormone. Auch eine gleichzeitige Bestimmung des bekannten Entzündungsparameters C-reaktives Protein (CRP) kann vorgesehen sein. Aufgrund der in dieser und den parallelen Anmeldungen beschriebenen neuen Ergebnisse ist für die Sepsis-Feindiagnose außerdem generell eine Kombination mit Messungen von bekannten oder noch aufzufindenden Biomolekülen in Betracht zu ziehen, die als gewebe- bzw. organspezifische Entzündungsmarker geeignet sind.

Epimerase oder ihre Bruchstücke oder Fusionsprodukte oder die dafür kodierende DNA können auch in der präventiven Medizin oder Therapie verwendet werden. So können z.B. geeignete Epimerase-Bruchstücke zur in-vivo-Erzeugung von Epimerase-bindenden Antikörpern durch aktive Immunisierung nach an sich bekannten Techniken verwendet werden. Als Epimerase sollen dabei auch solche Moleküle anzusehen sein, die die vollständige Epimerase oder geeignete Teilsequenzen davon in posttranslational modifizierter Form, z.B. in glykosylierter oder phosphorylierter Form, oder auch in einer durch pharmazeutische Hilfsstoffe, z.B. Polyethylenglykolreste, substituierten Form enthalten.

Epimerase oder geeignete Teilsequenzen davon können auch in dem Sinne als Target für therapeutische Interventionen dienen, dass mittels geeigneter spezifischer Binder für Epimerase oder Teilpeptide davon Epimerase intrakorporal inaktiviert wird oder ggf. auch extrakorporal im Sinne einer "Blutwäsche" bzw. Plasmapherese unter Verwendung geeigneter Immunadsorbentien bzw. unter Verwendung von mit spezifischen Bindern für Epimerase beschichteten perfundierbaren Festphasen aus dem Kreislauf von Patienten eliminiert wird. Wie in anderen bekannten Fällen, bei denen ein Krankheitsgeschehen durch Modifizierung der Wirkung und Aktivität daran beteiligter Enzyme beeinflusst wird, z.B. die bekannte Beinflussung von Infektions- und Erkältungskrankheiten durch Medikamente, die Cyclooxygenasehemmer darstellen, kann die Wirkung der A1E zu therapeutischen Zwecken außerdem auch medikamentös mit Hilfe von Arzneimitteln beeinflusst werden, die eine enzymhemmende, genauer A1E-hemmende, Wirkung aufweisen.

Zur in-vivo-Inaktivierung von Epimerase kommen neben Medikamenten insbesondere auch spezifische Antikörper, insbesondere humanisierte monoklonale Antikörper, in Frage. Die therapeutische Beeinflussung der Entzündungskaskade kann aber auch unter Einsatz der Epimerase selbst oder von Epimerase-Agonisten oder -Antagonisten erfolgen. Derartige therapeutische Interventionen werden insbesondere dann möglich, wenn weitere Erkenntnisse zur physiologischen Funktion der Epimerase im Rahmen eines Entzündungsgeschehens abgesichert sind. So erscheint es derzeit nicht ausgeschlossen, dass Epimerase im Entzündungsgeschehen eine wichtige Rolle spielt, möglicherweise durch direkte oder indirekte Beeinflussung des Krankheitsgeschehens durch Beteiligung an (De-)Glykosylierungsschritten und anderen Stoffwechselvorgängen bei der Aktiviereung oder Desaktiviereung von Peptiden, Proteinen, Lipiden, Zuckermolekülen und anderen Substanzen.

Nachfolgend wird die Auffindung und Identifizierung der Epimerase in näheren Einzelheiten geschildert, wobei auf das beigefügte Sequenzprotokoll bezug genommen wird. Die Figuren zeigen:
- Fig. 1: Ansichten von 2D-Elektrophoresegelen, die einen Vergleich der Spotmuster von cytoplasmatischen Leberzellprotein eines gesunden Pavians (A) mit den Leberzellproteinen eines Pavians 5h nach einer durch LPS-Verabreichung induzierten Sepsis (B) ermöglichen. Der Pfeil zeigt die Position des erfindungsgemäßen sepsisspezifischen Produkts Epimerase an, das in Darstellung (B) durch einen Kreis hervorgehoben ist;
- Fig. 2: das Massenspektrum des durch 2D-Gelelektrophorese separierten, isolierten und dann trypsinverdauten vollständigen Produkts A1E.
- Fig.3: das Tandem-Massenspektrum des einer ESI-MS-Analyse unterzogenen A1E-Fragments 710,3 aus Fig.2; und
- Fig.4: das Tandem-Massenspektrum des einer ESI-MS-Analyse unterzogenen A1E-Fragments 738,80 aus Fig.2.

### 1. Infektionssimmulation durch Endotoxinverabreichung im Tiermodell (Paviane).

In Anlehnung an die mit Pavianen durchgeführten Versuche zur Stimulierung der Procalcitonin-Ausschüttung durch Endotoxininjektionen (vgl.H.Redl, et al., "Procalcitonin release patterns in a baboon model of trauma and sepsis: Relationship to cytokines and neopterin", Crit Care Med 2000, Vol.28. No.11, 3659-3663; H.Redl, et al., "Non-Human Primate Models of Sepsis", in: Sepsis 1998; 2:243-253) wurden Pavianen (männlich, ca. 2 Jahre alt, 27 bis 29 kg schwer) jeweils 100 µg LPS (Lipopolysaccharid aus Salmonella Typhimurium, Bezugsquelle: Sigma) pro kg Körpergewicht intravenös verabreicht. 5 bis 5,5 h nach der Injektion wurden die Tiere durch intravenöse Verabreichung von 10 ml Doletal getötet. Innerhalb von 60 min nach ihrem Exitus wurden sämtliche Organe/Gewebe präpariert und durch Eintrieren in flüssigem Stickstoff stabilisiert.

Bei der weiteren Verarbeitung wurden Proben der einzelnen tiefgefrorenen Gewebe (1g) unter Stickstoffkühlung mit 1,5 ml Puffer A (50mM Tris/HCl, pH 7,1, 100mM KCl, 20% Glycerol) versetzt und in einem Porzellanmörser zu einem Mehl pulverisiert (vgl. J.Klose, "Fractionated Extraction of Total Tissue Proteins from Mouse and Human for 2-D Electrophoresis", in: Methods in Molecular Biology, Vol.112: 2-D Proteome Analysis Protocols, Humana Press Inc., Totowa, NJ). Nach einer anschließenden 1-stündigen Zentrifugation bei 100.000 g und +4°C wurde der erhaltene Überstand gewonnen und bis zur weiteren Verarbeitung bei -80°C gelagert.

Weil Versuche mit den wie oben gewonnenen Proben ergaben, dass die größte Procalcitoninmenge in Lebergewebe behandelter Tiere gefunden wird, wurde für die Suche nach neuen sepsisspezifischen Biomarkern mit Proteinextrakten aus der Pavianleber gearbeitet.

### 2. Proteomanalyse unter Verwendung cytoplasmatischer Leberzellproteine von Pavianen.

Cytoplasmatische Leberzellproteinextrakte von einerseits gesunden Pavianen (Kontrolle) und andererseits Pavianen, denen LPS injiziert worden war, wurden im Rahmen einer Proteomanalyse verwendet. Bei der einleitenden analytischen 2D-Gelelektrophorese wurde Leberextrakt, 100 µg Protein enthaltend, auf 9M Harnstoff, 70 mM DTT, 2% Ampholyt pH 2-4 eingestellt und dann mittels analytischer 2D-Gelelektrophorese aufgetrennt, wie in J.Klose, et al., "Two-dimensional electrophoresis of proteins: An updated protocol and implications for a functional analysis of the genome", Electrophoresis 1995, 16, 1034-1059; beschrieben ist. Die Sichtbarmachung der Proteine im 2D-Elektrophoresegel erfolgte mittels Silverstaining (vgl. J.Heukeshoven, et al., "Improved silver staining procedure for fast staining in Phast-System Development Unit. I. Staining of sodium dodecyl gels", Electrophoresis 1988, 9, 28-32).

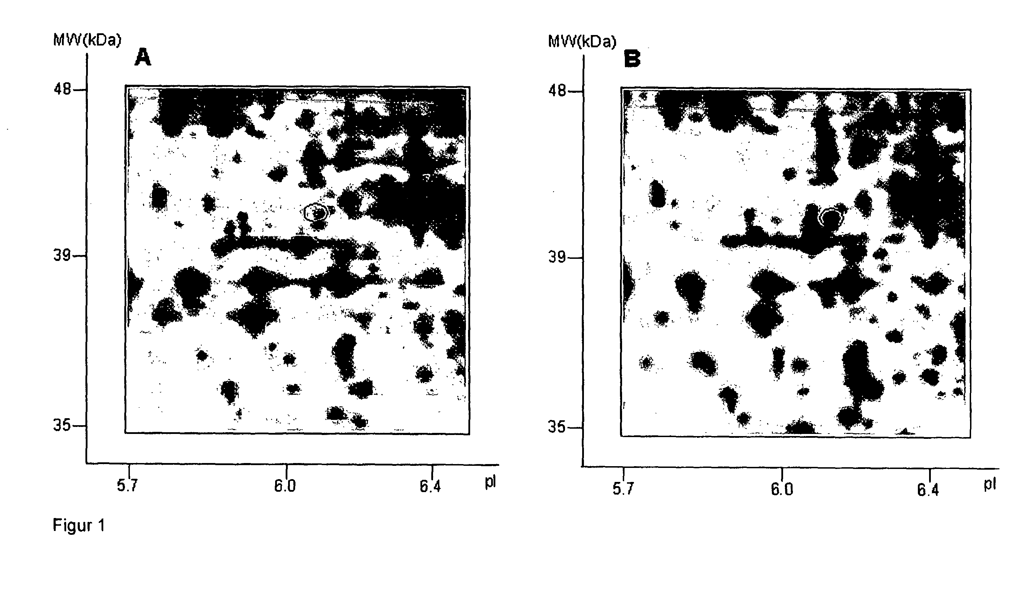

Zur Auswertung wurden die Proteinspotmuster der Proben unbehandelter Tieren mit den Proteinspotmustern verglichen, die aus Lebergewebeproben behandelter Tiere resultierten. Substanzen, die bei keiner Kontrollprobe, aber bei allen behandelten Tieren zusätzlich auftraten, wurden für weitere analytische Untersuchungen selektiert. Fig. 1 zeigt einen Vergleich der 2D-Elektrophoresegele für eine Kontrollprobe (A) und eine Probe eines behandelten Tieres (B), wobei der zusätzliche Proteinspot in (B) Epimerase entspricht, deren Position durch einen Pfeil und einen Kreis hervorgehoben ist.

Die im Proteinspotmuster der analytischen 2D-Gelelektrophorese identifizierten neuen spezifischen Proteine wurden dann anschließend mittels präparativer 2D-Gelelektrophorese unter Einsatz von 350 µg Protein präpariert (vgl. wiederum (10). Bei der präparativen 2D-Gelelektrophorese erfolgte die Färbung mittels Coomassie Brilliant Blue G250 (vgl. V.Neuhoff, et al., "Improved staining of proteins in polyacrylamide gels including isoelectric focusing gels with clear background at nanogram sensitivity using Coomassie Brilliant Blue G-250 and R-250", Electrophoresis 1988, 9, 255-262).

Der für die weitere Analyse vorselektierte Proteinspot wurde aus dem Gel ausgeschnitten. Er wurde unter Anwendung der Methode, die in A.Otto, et al., "Identification of human myocardial proteins separated by two-dimensional electrophoresis using an effective sample preparation for mass spectrometry", Electrophoresis 1996, 17, 1643-1650; beschrieben ist, trypsinverdaut und anschließend massenspektroskopisch analysiert und zwar unter Anwendung massenspektrometrischer Untersuchungen, wie sie z.B. in G.Neubauer, et al., "Mass spectrometry and EST-database searching allows characterization of the multi-protein spliceosome complex", in: nature genetics vol. 20, 1998, 46-50; J.Lingner, et al., "Reverse Transcriptase Motifs in the Catalytic Subunit of Telomerase", in: Science, Vol.276, 1997, 561-567; M.Mann, et al., "Use of mass spectrometry-derived data to annotate nucleotide and protein sequence databases", in: TRENDS in Biochemical Sciences, Vol.26, 1, 2001, 54-61; beschrieben und diskutiert werden. Dabei wurden die trypsinverdauten Proben nach einer ESI (ElectroSprayIonisierung) einer Tandem-Massenspektrometrie unterzogen. Es wurde ein Q-TOF-Massenspektrometer mit einer sog. nanoflow-Z-Spray-Ionenquelle der Firma Micromass, UK, verwendet. Dabei wurde entsprechend der Arbeitsanleitung des Geräteherstellers gearbeitet.

### 3. Identifizierung von Epimerase

Wie in den Figuren 1(A) und 1(B) gezeigt ist, findet sich in Leberzellextrakten von Pavianen, denen eine LPS-Injektion verabreicht worden war, u.a. ein neues Protein, für das aufgrund der Gelektrophoresedaten im Vergleich mit Markersubstanzen mit bekanntem Molekulargewicht ein Molekulargewicht von ca. 40000 ± 2000 Dalton abgeschätzt wurde, während aus der relativen Position des Proteins aus der ersten Dimension ein isoelektrischer Punkt von ca. 6 bis 6,4 abgeschätzt wurde.

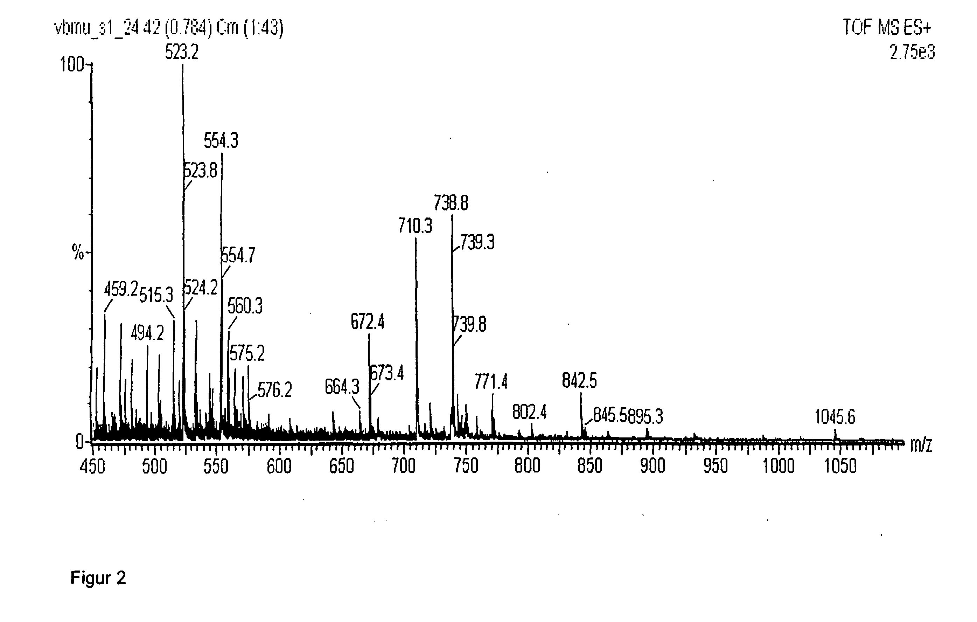

Dieses Protein wurde massenspektrometrisch analysiert, wobei Fig. 2 das Massenspektrum des gesamten trypsinverdauten Proteins zeigt.

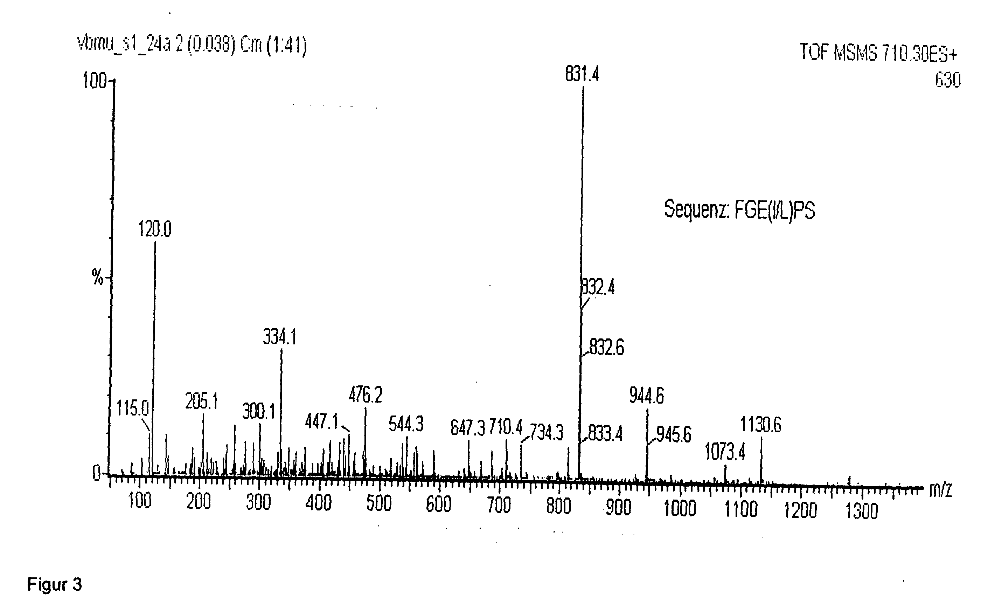

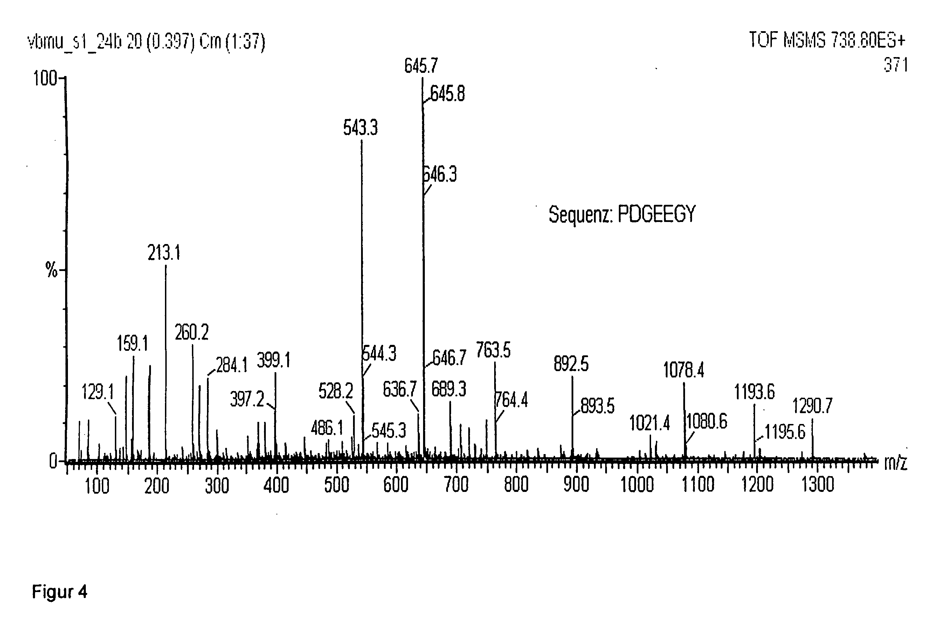

Fragmente des "Mutterspektrums" gemäß Fig. 2 wurden durch Tandem-Massenspektroskopie identifiziert. Die für zwei dieser Fragmente erhaltenen Massenspektren sind in den Fig. 3 und 4 geziegt. Die Fragmente konnten auf an sich bekannte Weise rechnerisch als die Peptid-Teilsequenzen SEQ ID NO:1 und SEQ ID NO:2 identifiziert werden.

Die durch Tandem-Massenspektrometrie identifizierten Teilsequenzen gemäß SEQ ID:NO:1 und SEQ ID NO:2 wurden dann mit Proteinsequenzen verglichen, die in Sequenzdatenbanken verfügbar waren. In der cDNA Datenbank NCBI, Accession No. AAH14916, wurde eine Aminosäuresequenz für ein hypothetisches menschliches Protein aus 342 Aminosäuren mit dem theoretischen Molekulargewicht von 37765 Dalton gefunden (vgl. SEQ ID NO:1), in der die Teilsequenzen gemäß SEQ ID NO:1 und SEQ ID NO:2 in den Positionen 9 bis 14 beziehungsweise 134 bis 140 enthalten waren. Durch Sequenzvergleich mit zwei mit NiceProt View of TrEMBL zu findenden Aminosäuresequenzen, von denen die eine humanen Ursprungs ist und 204 Aminosäuren umfaßt (Entry name/Primary accession No. Q12915) und die andere aus Schweinenieren stammt (Entry name/Primary accession No. Q9GKX6), und die beide der Aldose-1-Epimerase zugeordnet sind, konnte die Sequenz gemäß SEQ ID NO:3 als Aminosäuresequenz der humanen Aldose-1-Epimerase identifiziert werden.

Die damit explizit bekannte vollständige Sequenz der humanen Epimerase gestattet es, für humanmedizinische Zwecke (Diagnostik oder Therapie) humane Epimerase oder beliebige Teilsequenzen (Fragmente) davon gezielt herzustellen, und zwar unter Anwendung bekannter synthetischer oder gentechnologischer Verfahren zur Herstellung von Peptiden. Für veterinärmedizinische Zwecke gilt entsprechendes, wobei in diesen Fällen auf entsprechende bekannte, z.B. bovine, Epimerase-Sequenzen zurückgegriffen werden kann, oder tierspezifische Epimerase-Sequenzen in entsprechenden tierspezifischen Datenbanken aufgrund der Analogien mit den bekannten bovinen und humanen Sequenzen leicht aufgefunden werden können. Derartige Peptide können dann, beispielsweise in Analogie zu der in P.P.Ghillani, et al., "Monoclonal antipeptide antibodies as tools to dissect closely related gene products", The Journal of Immunology, vol. 141, No.9, 1988, 3156-3163; und P.P.Ghillani, et al., "Identification and Measurement of Calcitonin Precursors in Serum of Patients with Malignant Diseases", Cancer research, vol.49, No.23, 1989, 6845-6851; beschriebenen Arbeitsweise zur Schaffung von geeigneten Antikörpern, insbesondere monoklonalen Antikörpern dienen, die ihrerseits die Schaffung von Assays für die immundiagnostische Bestimmung der Epimerase oder ausgewählter Teilpeptide davon ermöglichen.

Auf die skizzierte bekannte Weise erhältliche monoklonale Antikörper können auch, insbesondere nach einer an sich bekannten Humanisierung, der Entwicklung von neuen Therapeutika dienen (vgl. die in K.Garber, "Protein C may be sepsis solution", Nature Biotechnology, Vol.18, 2000, 917-918; zusammengefassten therapeutischen Ansätze). Ferner ist eine in-vivo-Neutralisierung der Epimerase auch durch Blockade der Expression des Epimerase-Gens möglich. Zu den sich aufgrund der Erkenntnisse in der vorliegenden Anmeldung ergebenden therapeutischen Interventionen gehört auch die Verabreichung von Wirkstoffen, die die Enzymaktivität der Epimerase hemmen.

## Patentansprüche

1. Verwendung der Aldose-1-Epimerase (A1E) aus Körperflüssigkeiten oder Körpergeweben in der Human- und Tiermedizin als Markerpeptid zum diagnostischen Nachweis in vitro, für die Verlaufsprognose und für die Verlaufskontrolle von Entzündungen und Infektionen.

2. Verwendung von Aldose-1-Epimerase nach Anspruch 1 im Rahmen der differentialdiagnostischen Früherkennung und Erkennung, für die Verlaufsprognose, die Beurteilung des Schweregrads und für die therapiebegleitende Verlaufsbeurteilung von Sepsis und schweren Infektionen durch Bestimmung des Auftretens und/oder der Menge von Aldose-1-Epimerase in einer biologischen Flüssigkeit oder einer Gewebeprobe eines Patienten.

3. In vitro Verfahren zur differentialdiagnostischen Früherkennung und Erkennung, für die Verlaufsprognose und die Beurteilung des Schweregrads und zur therapiebegleitenden Verlaufsbeurteilung von Sepsis und schweren Infektionen, **dadurch gekennzeichnet, dass** man die Anwesenheit und/oder Menge von huamner Aldose-1-Epimerase (SEQ ID NO:3) in einer biologischen Flüssigkeit oder einer Gewebeprobe eines Patienten bestimmt und aus deren Nachweisbarkeit und/oder Menge Schlüsse hinsichtlich des Vorliegens, des zu erwartenden Verlaufs, des Schweregrads oder den Erfolg eines Therapie der Sepsis oder von Infektion zieht.

4. Verfahren nach Anspruch 3, **dadurch gekennzeichnet, dass** es ein immundiagnostisches Bestimmungsverfahren ist.

5. Verfahren nach Anspruch 3, **dadurch gekennzeichnet, dass** die Bestimmung der Aldose-1-Epimerase indirekt als Bestimmung der zugehörigen A1E-mRNA oder der A1E-Enzymaktivität erfolgt.

6. Verfahren nach einem der Ansprüche 3 bis 5, **dadurch gekennzeichnet, dass** es im Rahmen einer Multiparameter-Bestimmung durchgeführt wird, bei der gleichzeitig mindestens ein weiterer Sepsisparameter bestimmt wird und ein Messergebnis in Form eines Satzes von mindestens zwei Messgrößen gewonnen wird, der zur Sepsis-Feindiagnostik ausgewertet wird.

7. Verfahren nach Anspruch 6, **dadurch gekennzeichnet, dass** im Rahmen der Multiparameter-Bestimmung neben Aldose-1-Epimerase wenigstens ein weiterer Parameter bestimmt wird, der ausgewählt ist aus der Gruppe, die besteht aus Procalcitonin, CA 19-9, CA 125, S100B, S100A-Proteinen, löslichen Cytokeratin-Fragmenten, insbesondere CYFRA 21, TPS und/oder löslichen Cytokeratin-1-Fragmenten (sCY1F), den Peptiden Inflammin und CHP, Peptid-Prohormonen, der Glycin-N-Acyltransferase (GNAT) und dem C-reaktiven Protein (CRP).

8. Verfahren nach Anspruch 6 oder 7, **dadurch gekennzeichnet, dass** die Multiparameter-Bestimmung als Simultanbestimmung mittels einer Chiptechnologie-Messvorrichtung oder einer immunchromatographischen Messvorrichtung erfolgt.

9. Verfahren nach Anspruch 8, **dadurch gekennzeichnet, dass** die Auswertung des mit der Messvorrichtung gewonnenen komplexen Messergebnisses mit Hilfe eines Computerprogramms erfolgt.

## Claims

1. Use of aldose 1-epimerase (A1E) from body fluids or body tissues in human and veterinary medicine as a marker peptide for *in vitro* diagnosis, for prognosis of the course and for monitoring of the course of inflammations and infections.

2. Use of aldose 1-epimerase according to Claim 1 in differential early diagnosis and detection, for prognosis of the course, for assessment of the severity and for therapy-accompanying assessment of the course of sepsis and severe infections, by determination of the occurrence and/or of the amount of aldose 1-epimerase in a biological fluid or a.tissue sample of a patient.

3. *In vitro* method for differential early diagnosis and detection, for prognosis of the course and assessment of the severity and for therapy-accompanying assessment of the course of sepsis and severe infections, **characterized in that** the presence and/or amount of human aldose 1-epimerase (SEQ ID NO:3) in a biological fluid or a tissue sample of a patient is determined and conclusions are drawn from the detectability and/or amount thereof with regard to the presence, the expected course, the severity or the success of a therapy of the sepsis or of infection.

4. Method according to Claim 3, **characterized in that** it is an immunodiagnostic assay method.

5. Method according to Claim 3, **characterized in that** the determination of aldose 1-epimerase is carried out indirectly as a determination of the corresponding A1E-mRNA or of the A1E enzyme activity.

6. Method according to any of Claims 3 to 5, **characterized in that** it is carried out as part of a multiparameter determination in which at least one further sepsis parameter is simultaneously determined and a measured result in the form of a set of at least two measured variables, which is evaluated for fine sepsis diagnosis, is obtained.

7. Method according to Claim 6, **characterized in that**, in the course of the multiparameter determination, at least one further parameter which is selected from the group consisting of procalcitonin, CA 19-9, CA 125, S100B, S100A proteins, soluble cytokeratin fragments, in particular CYFRA 21, TPS and/or soluble cytokeratin 1 fragments (sCY1F), the peptides inflammin and CHP, peptide prohormones, glycine N-acyltransferase (GNAT) and the C-reactive protein (CRP) is determined in addition to aldose 1-epimerase.

8. Method according to Claim 6 or 7, **characterized in that** the multiparameter determination is carried out as a simultaneous determination by means of a chip technology measuring device or an immunochromatographic measuring device.

9. Method according to Claim 8, **characterized in that** the evaluation of the complex measured result obtained by means of the measuring device is carried out with the aid of a computer program.

## Revendications

1. Utilisation de l'aldose-1-épimérase (A1E) provenant de liquides corporels ou de tissus corporels en médecine humaine ou animale, comme peptide de marquage pour la détection in vitro en vue du diagnostic, du pronostic de l'évolution et du contrôle de l'évolution d'inflammations et d'infections.

2. Utilisation d'aldose-1-épimérase selon la revendication 1 pour la détection précoce par diagnostic différentiel et l'identification, pour le pronostic de l'évolution, l'évaluation du degré de gravité et l'évaluation de l'évolution conjointement à une thérapie de sepsies et d'infections graves, par détermination de la présence et/ou de la quantité d'aldose-1-épimérase dans un liquide biologique ou un échantillon de tissu d'un patient.

3. Procédé in vitro de détection précoce par diagnostic différentiel et d'identification, pour le pronostic de l'évolution, l'évaluation du degré de gravité et l'évaluation de l'évolution d'une sepsie et d'infections graves conjointement à une thérapie, **caractérisé en ce que** l'on détermine la présence et/ou la quantité d'aldose-1-épimérase (SEQ ID NO:3) humaine dans un liquide biologique ou un échantillon de tissu d'un patient, et **en ce qu'**à partir de la possibilité de sa détection et/ou à partir de sa quantité, on tire des conclusions sur la présence, l'évolution attendue, le degré de gravité et le résultat d'une thérapie de la sepsie ou de l'infection.

4. Procédé selon la revendication 3, **caractérisé en ce qu'**il s'agit d'un procédé de détermination par immunodiagnostic.

5. Procédé selon la revendication 3, **caractérisé en ce que** la détermination de l'aldose-1-épimérase s'effectue indirectement sous la forme d'une détermination de l'ARNm associé à l'AlE, ou de l'activité enzymatique de l'A1E.

6. Procédé selon l'une des revendications 3 à 5, **caractérisé en ce qu'**il est mis en oeuvre dans le cadre d'une détermination de plusieurs paramètres, dans laquelle on détermine en même temps au moins un autre paramètre de sepsie, et on recueille un résultat de mesure sous la forme d'un ensemble d'au moins deux grandeurs de mesure, qui est évalué pour fournir un diagnostic fin de la sepsie.

7. Procédé selon la revendication 6, **caractérisé en ce que** dans le cadre de la détermination de plusieurs paramètres on détermine, en plus de l'aldose-1-épimérase, au moins un autre paramètre qui est sélectionné dans le groupe qui est constitué de la procalcitonine, du CA 19-9, du CA 125, du S100B, des protéines S100A, des fragments solubles de cytokératine, en particulier le CYFRA 21, le TPS et/ou les fragments solubles de cytokératine-1 (sCY1F), des peptides inflammine et CHP, des prohormones peptidiques, de la glycine-N-acyltransférase et de la protéine C-réactive (CRP).

8. Procédé selon la revendication 6 ou 7,
**caractérisé en ce que** la détermination de plusieurs paramètres s'effectue sous la forme d'une détermination simultanée au moyen d'un dispositif de mesure basé sur la technologie des circuits intégrés, ou d'un dispositif de mesure d'immunochromatographie.

9. Procédé selon la revendication 8, **caractérisé en ce que** l'évaluation du résultat complexe de mesure obtenu avec le dispositif de mesure s'effectue à l'aide d'un programme informatique.
